# EUROPEAN PATENT APPLICATION

(11) **EP 2 080 518 A1**
(43) Date of publication of application: **22.07.2009**
(21) Application number: 08172113.6
(22) Date of filing: 18.12.2008
(51) Int. Cl.: A61K 36/24

(54) **Process for obtaining dried plant material**

(30) Priority: 18.01.2008 EP 08150389
(71) Applicant: Unilever N.V., 3013 AL Rotterdam (NL); Unilever PLC, 100 Victoria Embankment London, Greater London EC4Y 0DY (GB)
(72) Inventor: Dawes, Lindsay Vanessa, Boksburg 1460 Gauteng (ZA); Meeuse, Frederik Michiel, 3133 AT, Vlaardingen (NL)
(74) Representative: Wurfbain, Gilles L.

(57) **Abstract**

A process for obtaining dried plant material of plants from the *Apocynaceae* family, also known as *Asclepiadaceae* family, which contain steroidal glycosides having appetite suppressant activity. Said process covers (after harvesting and removing the roots a comminution step, a holding step of 1-36 hours in which moisture loss is limited, and a drying step.

## Description

### TECHNICAL FIELD

The present invention relates generally to the field of steroidal glycosides. More in particular, it relates to a process for obtaining dried plant material of plants from the *Apocynaceae* family, also known as *Asclepiadaceae* family, which contain steroidal glycosides having appetite suppressant activity and which can be used, for example, in weight management products. The invention especially relates to obtaining dried plant material of plants from the *Hoodia* genus (formerly the *Hoodia* and *Trichocaulon* genera).

### BACKGROUND OF THE INVENTION

Extracts obtainable from plants of the *Apocynaceae* family, also known as *Asclepiadaceae* family, particularly the *Hoodia* genus (formerly the *Hoodia* and *Trichocaulon* genera) have been shown to have an appetite suppressant activity and are potentially useful in weight management products. US 6,376,657 (CSIR) discloses that these extracts contain steroidal glycosides having the formula 1 : 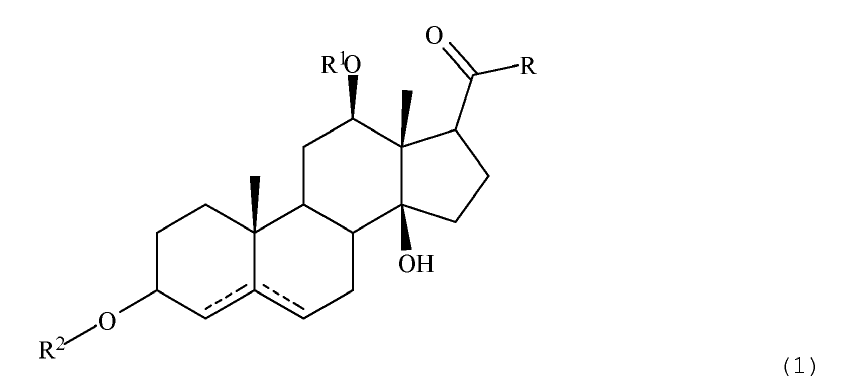 wherein
R = alkyl;
R¹ = H, alkyl, tiglyol, benzoyl or any other organic ester group;
R² = H or one or more 6-deoxy carbohydrates, or one or more 2,6-dideoxy carbohydrates, or glucose molecules, or combinations thereof; and wherein the broken lines indicate the optional presence of a further bond between carbon atoms C4 and C5 or between carbon atoms C5 and C6.

The active molecules in purified fractions having good appetite supressant activity (and of which molecules the structure has been identified) were found to be compounds having the formula (2) to (8) (Me=CH₃) :

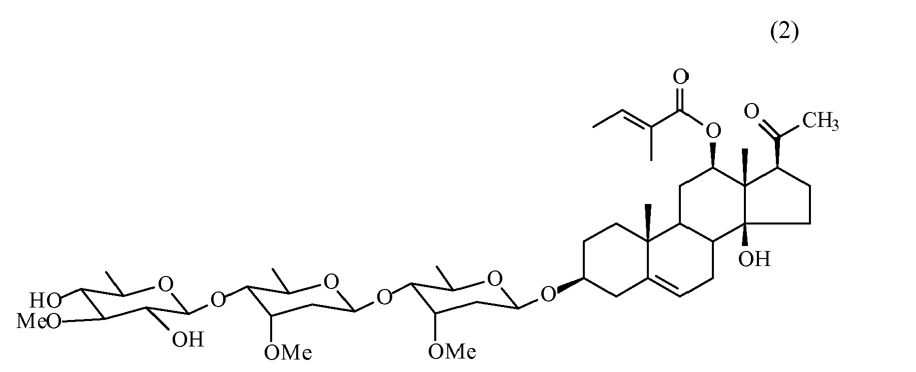

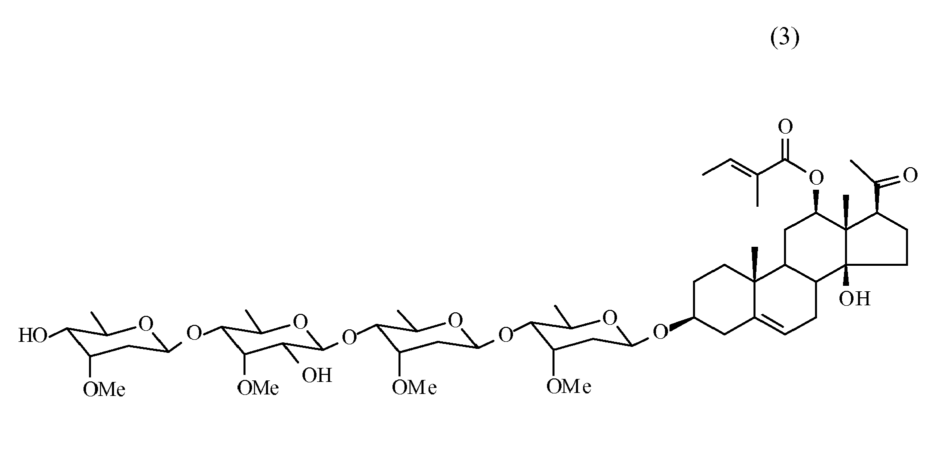

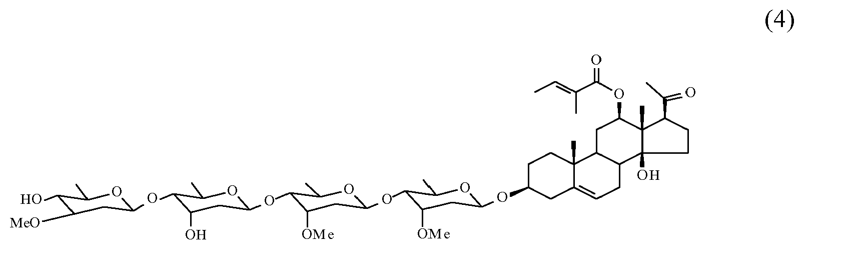

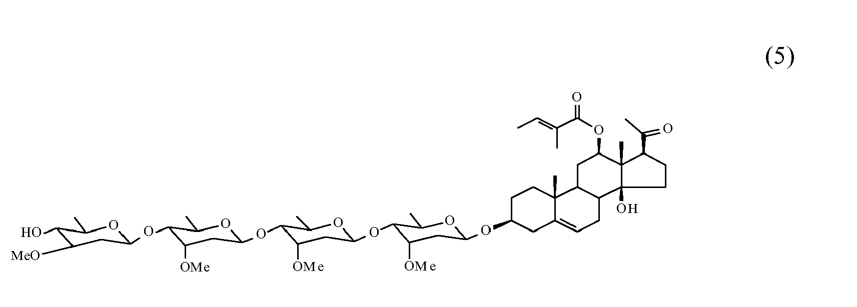

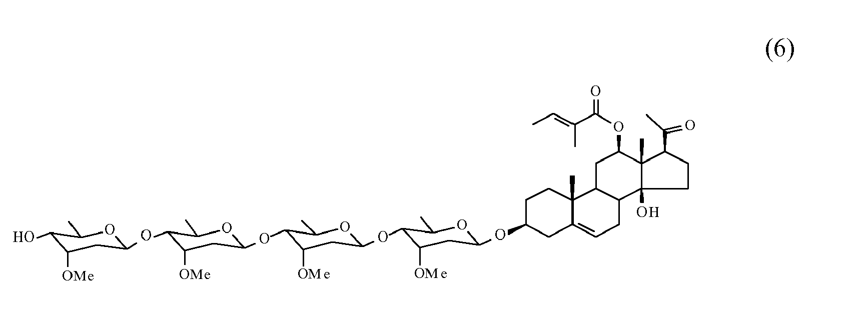

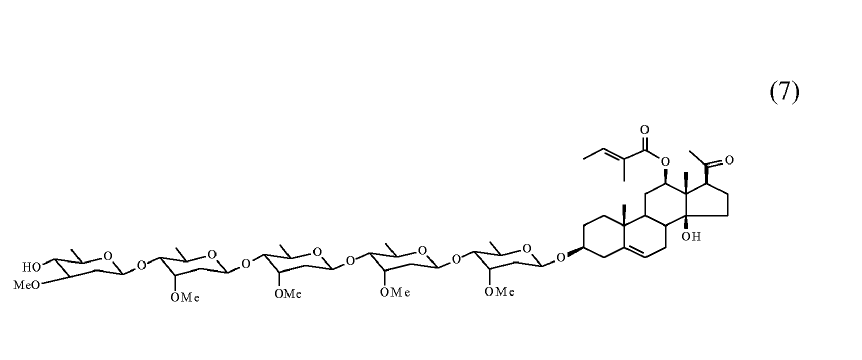

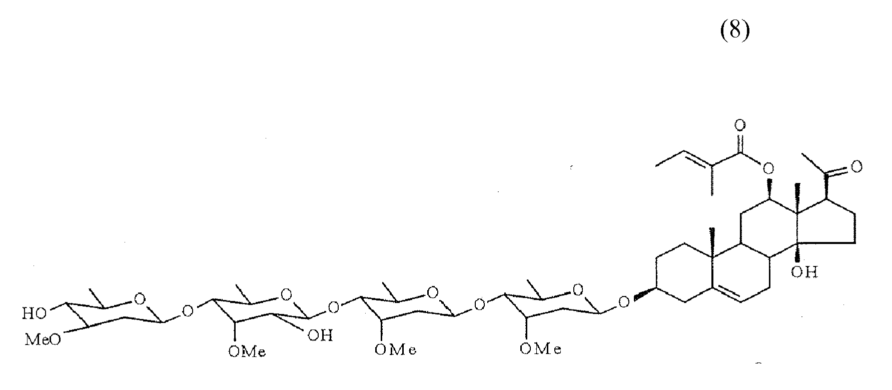

US 6,376,657 also discloses a process to extract the steroidal glycoside having the formula 1 from plants of the *Asclepiadaceae* family, involving treating plant material with a solvent to extract a fraction having appetite suppressant activity, separating the extraction solution from the rest of the plant material, removing the solvent from the extraction solution and recovering the extract.
The patent also discloses methods for synthesizing various steroidal glycosides.

WO2005/116049 discloses that steroidal glycosides can be extracted or separated from undesirable components present in plant material of the *Asclepiadaceae* (Hoodia) family by means of liquid or supercritical carbon dioxide. Dried plant material from *Hoodia gordonii* was milled to a fine powder and subsequently extracted.

US 2005/0202103 discloses *Caralluma* extracts, wherein the aerial parts of Caralluma plant are dried under shade (on cemented platform).

US 7,008,648 discloses a method of obtaining a plant material from Stapelia and Orbea plants, wherein a suitable method for drying and grinding the original biomass includes either sun drying followed by a heated air-drying or freeze-drying, e.g. lyophilization or chopping of the biomass into small pieces, e.g. 2-10 cm, followed by heated air-drying or freeze-drying.

Patent application PCT/EP2007/057813 relates to a process for obtaining dried plant material from plants of the family the *Apocynaceae*, wherein the process comprises the steps of harvesting the plants, cutting up the harvested plants, followed by drying said cut plant material at conditions which comprise a limited amount of UV exposure during drying. This case shows that drying in an oven for 48 hours at 70°C was the drying method with the highest level of the desired actives. However, a drying process of 48 hours has economical disadvantages, e.g. in that drying equipment is needed which can hold a high amount of material to be dried.

US 7,265,101 discloses that the conditions under which plants are grown may affect the appetite-suppressing compound content of plants of the genus *Asclepias*.

US 7,008,648 discloses a method for obtaining appetite-suppressing material from plants of the genera Stapelia and *Orbea* (the latter also referred to as *Asclepiadaceae*).

### SUMMARY OF THE INVENTION

The purification and isolation of steroidal glycosides having appetite suppressant effects, especially those of formulas (2) to (8) (for which an appetite supressing effect is proven and which compounds have been identified), from the plants of the Apocynaceae family is costly and cumbersome. It is therefore desirable to improve the yield of (or the manufacture of a preparation, e.g. a vegetable or plant preparation, rich in) steroidal glycosides, especially the steroidal glycosides having one of the formulas (2) to (8).

A process starting with harvesting the plants of the Apocynaceae family for the purpose of obtaining a composition rich in one or more of the actives of formulas (2) to (8) in such a way that edible products and food products can easily be prepared that contain such actives usually involves a drying step, in which the plant material is dried to a moisture content of e.g. below 10% (preferably below 5%, by weight). Preferably, for reasons of economy, this drying step is performed quickly. It was found, however, that if the drying is done quickly (e.g. in less than 3 hours at 70°C) the level of actives, in particular of the desired actives (2) to (8) drops, when compared to slow drying (e.g. 48 hours, 70°C).

It is known that the plants of the *Apocynaceae* family do not only contain the identified steroidal glycosides (2) to (8) which are known or reported to have appetite-suppressing properties and for which the structure has been identified, but also that such plants contain other steroidal glycosides. Such "other steroidal glycosides" may be obtained in admixture with the known actives of formulas (2) to (8). Such "other steroidal glycosides" may also be active as appetite suppressing compound, or may not be active: efficacy has not been proven and/or the structure has not been identified. Such "other steroidal glycosides" (or rather the way in which they can be obtained) can be identified as peaks in a HPLC method as described in more detail herein below. As such other steroidal glycosides may have undesired properties (e.g. bitterness) or at least unknown properties, and the efficacy of them in appetite suppression is unknown, the level of such "other steroidal glycosides" is preferably kept low, when enhancing the level of the desired, known, and proven compounds of formulas (2) to (8). Formulated in another way, it is desired that one can keep the ratio of desired steroidal glycosides (2) to (8) taken together to "other steroidal glycosides" (e.g. in dried plant material) above a ratio of 0.4.

Hence, there was a need for a process for obtaining dried plant material of the the *Apocynaceae* family, which process involves a drying step to obtain a material comprising one or more of the components of formulas (2) to (8) and which material has a moisture content of below 10% (preferably a moisture content of 0.5-10%, more preferably 1-8% by weight), and for which process the actual drying step (i.e. from a material which contains more than 90% of its moisture compared to when measured directly after harvesting, to a material which contains e.g. less than 10% total moisture), takes less than 12 hours, preferably less than 8 hours, and for which process still good levels of actives of one or more of formulas (2) to (8) are resulting in the end product (the moisture loss can be e.g. by weight loss, when compared to the fresh harvested plant material). Regarding this, it is preferred that said plant material should contain less than 10% moisture and component (2) to (8) taken together in a (mean) amount of at least 0.35% by weight (based on the anhydrous plant material), preferably at least 0.4% by weight. Furthermore, it is desired that in the dried vegetable or plant material the (mean) weight ratio between the steroidal glycosides of formulas (2) to (8) (when taken together) to other steroidal glycosides is at least 0.4, more preferably said ratio is at least 0.45.

It has now been found that such can conveniently be achieved (at least in part) by a process for obtaining dried plant material from the *Apocynaceae* family comprising the steps of:
(a) removing the plants from the soil,
(b) cutting up the plants to pieces,
(c) holding the cut up plants for 1 - 36 hours, preferably 2-24 hours, at a temperature of 15-40°C wherein weight loss during this holding step is 0-10% of the fresh cut material,
(d) drying the so-obtained cut up plants, at a temperature of 40-120°C for 0.5 to 12 hours to arrive at a moisture content of below 10% (by weight).

### DETAILED DESCRIPTION OF THE INVENTION

Except in the operating and comparative examples, or where otherwise explicitly indicated, all numbers in this description indicating amounts of material or conditions of reaction, physical properties of materials and/or use are to be understood as modified by the word "about".

It should be noted that in specifying any range of concentration or amount, any particular upper concentration can be associated with any particular lower concentration or amount.

For the avoidance of doubt the word "comprising" is intended to mean "including" but not necessarily "consisting of" or "composed of". In other words, the listed steps or options need not be exhaustive.

"Cut" as used herein means that the size of the plant is reduced, and includes comminuting, pulverising, etc.

"Mean" as used herein means the average steroidal glycoside content of at least 10 different, randomly selected, plants.

The steroidal glycosides of formulas (2) to (8) have been proven to be effective as appetite supressing compound. Steroidal glycoside concentrations are determined using high performance liquid chromatography (HPLC) with UV detection after extraction or dissolution.
In case of dried plant material approximately 5 g of material is refluxed with approx. 80 ml of boiling methanol for 1 hour. The resulting extract is filtered and the solid material is washed with methanol. The combined filtrate and washing are transferred to a 100 ml flask and made to volume with methanol. 1 ml of the filtrate is evaporated to dryness and reconstituted in 1 ml acetonitrile/water (50/50 v/v).

The steroidal glycosides are measured by LC-UV at 220 nm. To this end 20 µl of the extracts are injected onto a Zorbax RX-C8 analytical column of 250 x 4.6 mm packed with 5 µm particles and equipped with a Zorbax RX-C8 guard column of 12.5 x 4.6 mm packed with the same stationary phase. The column system is held at 40°C. Gradient elution is performed starting at 41.2% acetonitrile/methanol (85/15 v/v) and 58.8% water/methanol (85/15 v/v) at a flow rate of 1 ml/min. Initial conditions are held for 10 minutes before being linearly increased to 88.2% acetonitrile/methanol (85/15 v/v) and 11.8% water/methanol (85/15 v/v) over 30 minutes. After a final hold of 5 minutes the system is re-equilibrated to the starting conditions.

Compound of Formula 2 of any known purity (95% was used in this case) is used for calibration. Compound 2 may be isolated from an extract of dried *Hoodia* gordonii using preparative liquid chromatography or may be synthesized (see e.g. US Patent 6,376,657, incorporated by reference herein). A stock solution at 100 µg/ml is prepared in acetonitrile/water (1/1 v/v) and further dilutions are prepared to yield additional calibration standards at 75, 50, 20, 10 and 5 µg/ml. UV response at 220 nm is used for quantification against the Compound 2 calibration line. Relative response factors based on molecular weight are used to quantify the steroidal glycosides against the Compound 2 calibration line. Steroidal glycosides are defined as all peaks eluting between 15 and 45 minutes that were not present in the blank acetonitrile/water (1/1 v/v) sample. This group of steroidal glycosides eluted in said time interval can be divided in the identified actives of formulas (2) to (8) and the group of compounds eluted in the same time frame but which are not of the formulas (2) to (8). This second group is herein referred to as "other steroidal glycosides". Among such "other steroidal glycosides" may be components which are also active in appetite suppression, or they may be inactive: they have not been identified and/or studied.
The specific relative retention times and response factors, are summarized in Table 1.

**TABLE 1**

| Relative retention times and response factors of some steroidal glycosides | | |
|---|---|---|
| Compound | Relative retention time vs. Compound 2 | Response factor vs. Compound 2 |
| formula 2 | 1.000 | 1.000 |
| formula 8 | 1.066 | 1.164 |
| formula 3 | 1.128 | 1.164 |
| formula 4 | 1.191 | 1.130 |
| formula 5 | 1.292 | 1.146 |
| formula 6 | 1.328 | 1.146 |
| formula 7 | 1.399 | 1.309 |

The other steroidal glycosides peaks eluting after 15 minutes have a response factor of 1.081 vs. compound formula (2).

Thus, "steroidal glycoside" as used herein means a steroid (four fused rings), further comprising at least one side group substitution which is a glycoside (a molecule in which a sugar group is bonded through its anomeric carbon to another group via an O-glycosidic bond), preferably a deoxy or di-deoxy glycoside and includes all steroidal glycosides eluting between 15 and 45 minutes as described in HPLC Steroidal Glycoside Analysis above.

The first aspect of the present invention is a process for obtaining dried plant material of plants of the the *Apocynaceae* family, more preferably the *Hoodia* family. It is especially preferred if the plant is selected from the group consisting of *Trichocaulon piliferum, Trichocaulon officinale, Hoodia currorii, Hoodia gordonii, Hoodia lugardii* and mixtures thereof. *Hoodia gordonii* is especially preferred.

In the first step of the process, the plants of the *Apocynaceae* family are completely removed from the soil on which they were grown, preferably including the roots. This can be done either manually by pulling the plants out of the ground (possibly with help of e.g. a spade), or in an automated way, using a suitable harvesting machine or tool.

After removing the plants from the soil, the cured plants are subsequently cut. The plants can be cut into any shape, like cubes, slices, julliene, etc. as long as one of the dimensions of the so-obtained pieces is less then 30 mm, preferably less then 20 mm, most preferably less then 15 mm. So for a slice or julliene shape the thickness should be less than these dimensions, for a cube all dimensions should be less then these dimensions, etc. Coventional cutting equipment may be used, such as a wood chipper, a bowl cutter or standard food cutting equipment, for example the machines supplied by Urschell, to form cut plant particles. The smaller the size, the faster the subsequent drying time, reducing the possibility of microbial growth. For the cutting step and subsequent drying step, the whole plants may be used, but preferably the plants are used without roots, to mimize the possibility of microbial contamination. Preferably, in the process according to this invention, after harvesting the roots of the plants are removed from the plant prior to or simultaneously with cutting up the plants in step (b). Prior to cutting, the plants are preferably washed.

Following the cutting step, the cut plant particles are held (step (c) in the process according to the invention) for a time between 1 and 36 hours, preferably 2-24 hours, more preferably 4 to 16 hours. The temperature during this holding step is preferably 15-40°C, more preferably the temperature during holding step (c) is 20-35°C. Although some moisture loss of the cut vegetable matter may occur, such is preferably limited. Moisture loss will result in a loss of weight (evaporation of some moisture), and the holding step is preferably such that the weight loss occurring during the holding step is less than 10% of the weight of the fresh cut material (herein to be understood as after harvesting and after the root has been cut off). Preferably such weight loss during this holding step is 0-10% of the weight of the fresh cut material, more preferably 0.5-8%, even more preferably 1-5%. The holding of the material can be carried out in any suitable way which ensures the limited moisture loss in the given time and at the given temperature. Although the material may be spread out on the surface of a factory floor, it is more convenient to store the fresh cut matter in big storage containers, hoppers, silo's, bags, etcetera. This will both limit moisture loss and and is both convenient in terms of handling and space required. The storage containers may be open. Preferably, the holding is carried out in the shade, e.g. in containers (open or closed) which are stored inside a warehouse or factory or storage room, or under a roof. The storage may be conveniently carried out at ambient temperature. Preferably, no heating is applied of the material, and the material is thus preferably left to ambient temperature. Preferably, the material to be held is not exposed to forced air flow: such would both raise costs and may reduce the moisture content too much for the holding step.

It was surprisingly found that the holding step can yield similar levels of the desired steroidal glycosides or higher and allows subsequent quick drying, as when the material is subjected to slow drying. Thus, when the drying can be carried out, drying equipment (e.g. ovens) can be used which does not need to hold huge quantities for 24 to 48 hours. Simply holding the matter in a simple storage container under the conditions as set out herein, followed by relatively quick drying is sufficient to ensure a good level of the desired steroidal glycosides. Preferably, the drying step (d) is done at a temperature of 60-100°C, preferably at 70-90°C.

Following the holding step, held plant matter is subjected to the actual drying step (step (d) in the process herein) to yield a material which has a moisture content of below 10% (by weight). Preferably, such drying is done under such conditions whereby direct exposure to UV light is minimized.

Suitable drying equipment according to the present invention includes direct and indirect air dryers where the air is heated with any kind of energy source (e.g. electricity, gas, parafin, energy, etc.). Solar energy heats the air with the sun; the hot air may then be blown into an oven where the material is dried. "Drying" as used herein may include freeze-drying.

Typically, the drying step (d) is conducted at a temperature of from 40 to 120°C, preferably, in order to have optimum drying time, from 60 to 100°C, most preferably from 70 to 90°C. The drying period is typically 0.5-12 hours, preferably 1-8 hours, more preferably 1-5 hours.

In the drying step, the cut (and held) plant matter is typically dried to a residual moisture content of less than 10% by weight, preferably 0.5-10% by weight, more preferably 1-8% by weight. The (residual) moisture content can be measured using standard gravimetric techniques or Karl Fischer titration.

The process according to the present invention now enables the manufacture of a dried plant material which is relatively high in the desired steroidal glycosides. Hence, the present invention further relates to a process wheren the dried plant material further comprises one or more steroidal glycosides having the formulas (2) to (8) and mixtures thereof (Me=CH₃) :

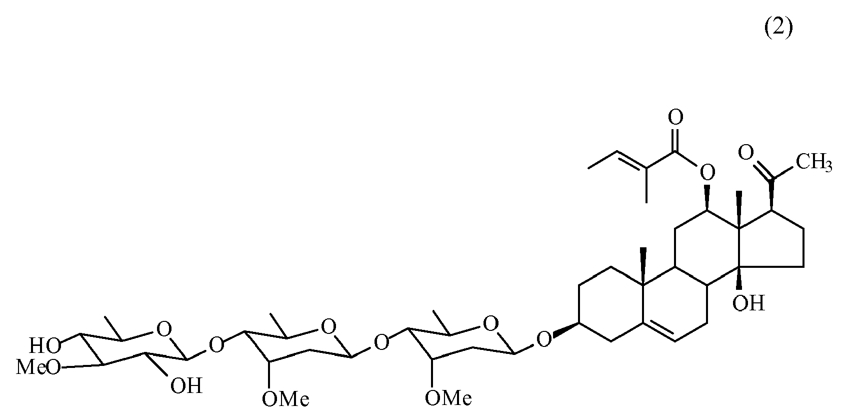

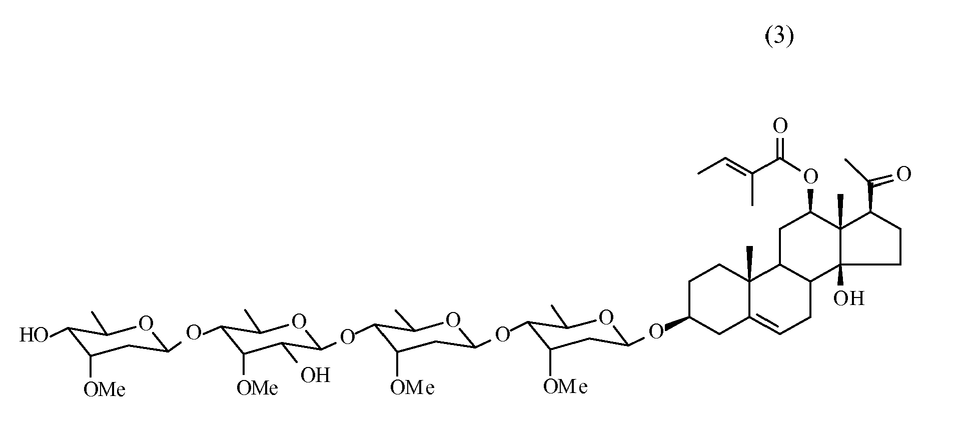

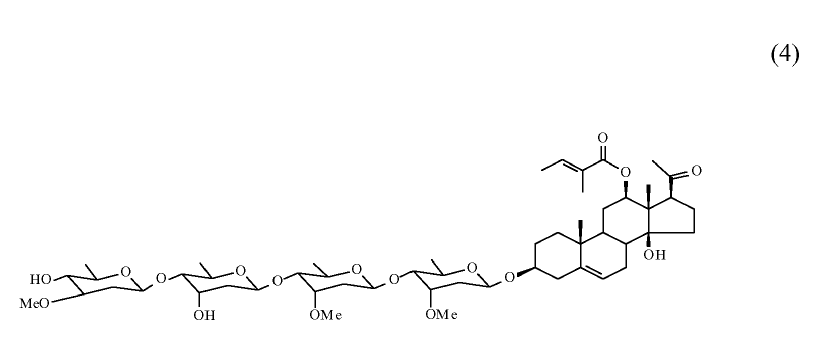

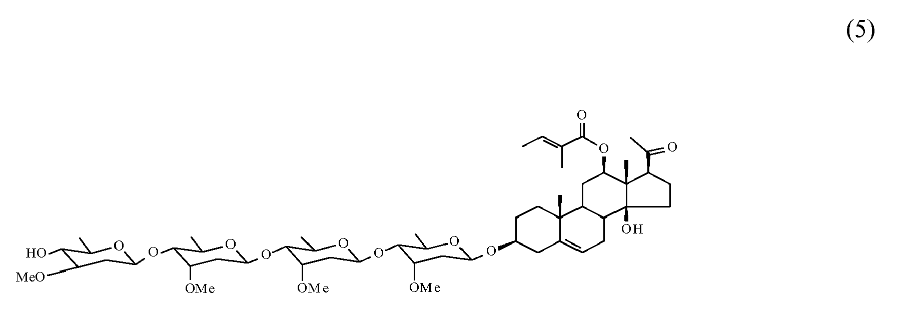

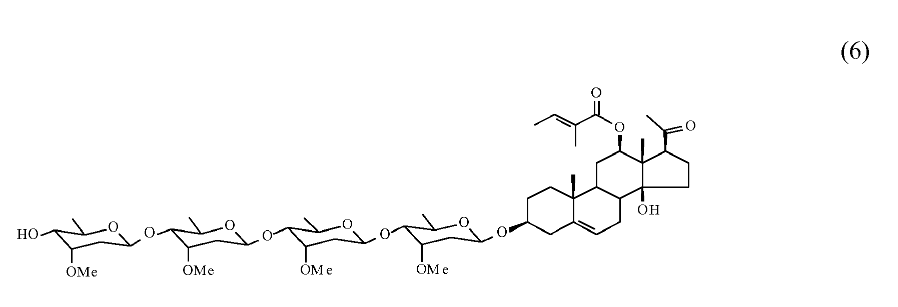

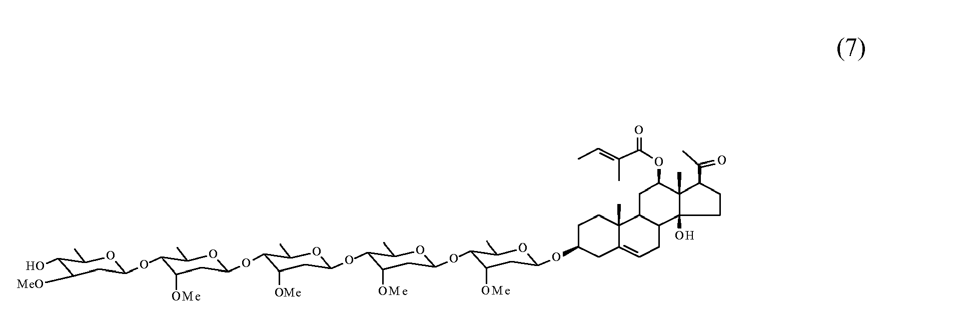

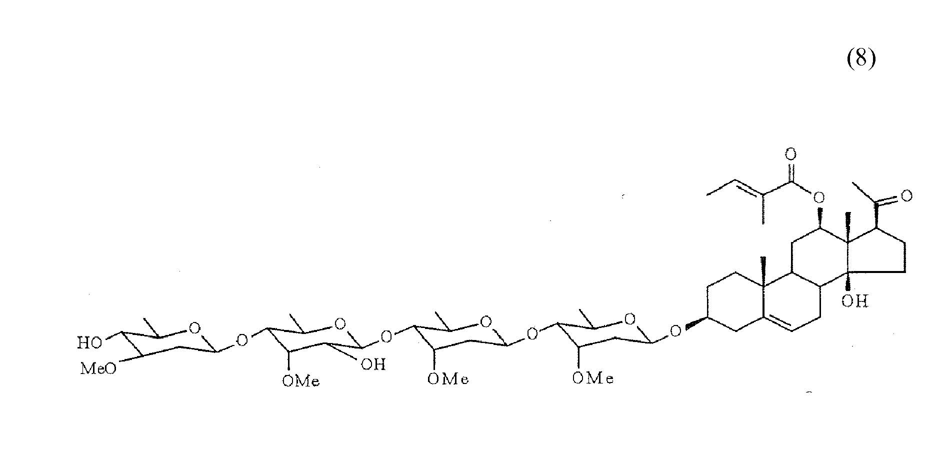

Preferably, such dried plant material has a mean steroidal glycoside content of compounds (2) to (8) taken together of at least about 0.35% by weight, preferably at least 0.4%, based on anhydrous dried plant material (i.e. 0% moisture).

The obtained dried plant material, preferably in the form of small pieces or flakes, has a mean total content of steroidal glycosides of at least 1.3% by weight, preferably of at least 1.6% by weight (the desired steroidal glycosides (2) to (8) and the other steroidal glycosides taken together).

The present invention further relates to the plant material obtainable by the process of this invention. Hence, in a further aspect the present invention relates to dried plant material obtainable by the process as set out herein, wherein at least one of the steroidal glycosides (2) to (8) is increased by at least 20% in weight compared to a plant material which is prepared by a process without step (c), and which dried plant material has a moisture content of below 10% by weight.

Likewise, the invention further relates to a composition comprising one or more of the steroidal glycosides of formulas (2) to (8) and "other steroidal glycosides", wherein the (mean) weight ratio between the steroidal glycosides of formulas (2) to (8) (when taken together) to "other steroidal glycosides" is at least 0.4, more preferably said ratio is at least 0.45. Preferably, the composition having such ratio between steroidal glycosides of formulas (2) to (8) and "other steroidal glycosides" is dried (0-10% moisture, preferably 3-8% moisture) comminuted plant material.

The dried plant material obtainable according to the invention comprises the steroidal glycoside of formula (2) in an amount of at least 0.095% by weight (calculated as a mean), preferably at least 0.1% by weight.

The amount of steroidal glycoside of Formula 2 in the dried plant material can be determined using high performance liquid chromatography (HPLC) with UV detection after extraction or dissolution. Approximately 5 g of material is refluxed with approximately 80 ml of boiling methanol for 1 hour. The resulting extract is filtered and the solid material is washed with methanol. The combined filtrate and washing are transferred to a 100 ml flask and made to volume with methanol. 1 ml of the filtrate is evaporated to dryness and reconstituted in 1 ml acetonitrile/water (50/50 v/v).

Steroidal glycosides are measured by LC-UV at 220 nm. To this end 20 µl of the extracts are injected onto a Zorbax RX-C8 analytical column of 250 x 4.6 mm packed with 5 µm particles and equipped with a Zorbax RX-C8 guard column of 12.5 x 4.6 mm packed with the same stationary phase. The column system is held at 40°C. Gradient elution is performed starting at 41.2% acetonitrile/methanol (85/15 v/v) and 58.8% water/methanol (85/15 v/v) at a flow rate of 1 ml/min. Initial conditions are held for 10 minutes before being linearly increased to 88.2% acetonitrile/methanol (85/15 v/v) and 11.8% water/methanol (85/15 v/v) over 30 minutes. After a final hold of 5 minutes, the system is re-equilibrated to the starting conditions.

Compound of formula (2) of any known purity (95% was used in this case) is used for calibration. Compound formula (2) may be isolated from an extract of dried *Hoodia* gordonii using preparative liquid chromatography or may be synthesized (see e.g. US Patent 6,376,657, incorporated by reference herein). A stock solution at 100 µg/ml is prepared in acetonitrile/ water (1/1 v/v) and further dilutions are prepared to yield additional calibration standards at 75, 50, 20, 10 and 5 µg/ml. UV response at 220 nm is used for quantification against the Compound 2 calibration line.

In a further embodiment of the process of the present invention, one or more steroidal glycosides are extracted from the dried plant material. Any extraction method may be employed. For instance extraction may be conducted as described in US 6,376,657, incorporated by reference herein. The solvents specifically mentioned to perform the extraction are one or more of methylene chloride (dichloromethane), water, methanol, hexane, ethyl acetate or mixtures thereof. Alternatively, the steroidal glycosides may be extracted using liquid or supercritical carbon dioxide such as described in W02005/116049.

Optionally, after the drying step an extraction step may be employed, to yield a material having an even higher amount of the desired steroidal glycosides. Hence, it may be preferred that the process according as set out herein further comprises a step (e) after (d), comprising the step of obtaining a fraction of the dried material which contains compounds of formulas (2) to (8). More preferably the process according as set out herein comprises a step (e) after (d), comprising the step of obtaining a fraction of the dried material which comprises compounds of:
formula (2) in a (mean) amount of 6-12%, formula (3) in a (mean) amount of 5-10%, formula (4) in a (mean) amount of 4-8%, formula (5) in a (mean) amount of 4-9%, formula (6) in a (mean) amount of 2-9%, formula (7) in a (mean) amount of 1-3%, formula (8) in a (mean) amount of 2-5%, after extraction of the dried plant material. Such extraction may suitably be carried out using the method as is set out in International Patent Application PCT/EP2007/057320.

The dried plant material or the extract therefrom can be used in appetite suppressant food products, and this constitutes a third aspect of the present invention. Examples of such food products are beverages, snacks, bars, spreads, dressings, soups, etc., or meal replacement products, which can be used in the management of body weight or in the dietary control of obesity.

All amounts, parts, ratios and percentages used herein are by weight, unless otherwise specified.

While the above summarizes the present invention, it will become apparent to those skilled in the art that modifications, variations and alterations may be made without deviating from the scope and spirit of the present invention as described and claimed herein. The invention will now be further illustrated in the following, non-limiting example.

### EXAMPLES

*Hoodia gordonii* plants (2 years old, grown in the Northern Cape province of South Africa) were harvested by tearing them out of the soil. The roots were cut off, almost directly after harvesting. After this, the so-obtained plants were transported in-doors, and comminuted (including stems and leaves) to particles having a size of about 10x10x6 mm by using an Urschell cutter.

The so-obtained plant particles were then stored in a big container, under ambient conditions in-doors (temperature varying between 20 and 25°C), for 3 different time intervals: 4, 12 and 16 hours. Such container (a sort of crate containing about 370 kg of cut *Hoodia gordonii* plant material) was open at the top, contained multiple holes on the side, but of smaller dimension than the cut *Hoodia gordonii* particles and covering less than 30% of the container wall surface. After storing for the specified times the moisture loss was less than 10% by weight based on the fresh weight. After such holding, the material was dried with a Proctor belt dryer at 80°C to a moisture content of 3-8% (wt). In addition to this, one sample was not stored but dried in the same way directly after cutting. The latter thus acts as a control, in which quick drying is employed (holding time 0 hours).

After drying, the four samples (holding time 0, 4, 12 and 16 hours) were analysed for the content of certain steroidal glycosides in the dried plant material: 7 desired ones, identified as (2) to (8), being the same structures as (2) to (8) in the patent specification herein, and a group "others". "Others" covers other steroidal glycosides as described above.

The content and identity of the steroidal glycoside concentrations was determined using high performance liquid chromatography (HPLC) with UV detection after extraction or dissolution. Approximately 5 g of dried plant material was refluxed with approx. 80 ml of boiling methanol for 1 hour. The resulting extract was filtered and the solid material was washed with methanol. The combined filtrate and washing were transferred to a 100 ml flask and made to volume with methanol.
1 ml of the filtrate was evaporated to dryness and reconstituted in 1 ml acetonitrile/water (50/50 v/v).

The steroidal glycosides were measured by LC-UV at 220 nm. To this end 20 µl of the extracts were injected onto a Zorbax RX-C8 analytical column of 250 x 4.6 mm packed with 5 µm particles and equipped with a Zorbax RX-C8 guard column of 12.5 x 4.6 mm packed with the same stationary phase. The column system was held at 40°C. Gradient elution was performed starting at 41.2% acetonitrile/methanol (85/15 v/v) and 58.8% water/methanol (85/15 v/v) at a flow rate of 1 ml/min. Initial conditions were held for 10 minutes before being linearly increased to 88.2% acetonitrile/methanol (85/15 v/v) and 11.8% water/methanol (85/15 v/v) over 30 minutes. After a final hold of 5 minutes the system was re-equilibrated to the starting conditions.

Compound of formula (2) of a known purity (95% was used in this case) was used for calibration. A stock solution at 100 µg/ml was prepared in acetonitrile/water (1/1 v/v) and further dilutions were prepared to yield additional calibration standards at 75, 50, 20, 10 and 5 µg/ml. UV response at 220 nm was used for quantification against the compound formula (2) calibration line. Relative response factors based on molecular weight were used to quantify the steroidal glycosides against the compound formula (2) calibration line. Steroidal glycosides were defined as all peaks eluting between 15 and 45 minutes that were not present in the blank acetonitrile/water (1/1 v/v) sample. This group of steroidal glycosides eluted in said time interval covered the identified actives of formulas (2) to (8) and the group of compounds eluted in the same time frame but which are not actives of the formulas (2) to (8). This second group was herein referred to as "other steroidal glycosides". Among such "other steroidal glycosides" may be components which are also active in appetite suppression, or they may be inactive: they have not been all identified nor all been studied. The specific relative retention times and response factors, are summarized in Table 2.

**TABLE 2**

| Relative retention times and response factors of some steroidal glycosides | | |
|---|---|---|
| Compound | Relative retention time vs. Compound 2 | Response factor vs. Compound 2 |
| formula 2 | 1.000 | 1.000 |
| formula 8 | 1.066 | 1.164 |
| formula 3 | 1.128 | 1.164 |
| formula 4 | 1.191 | 1.130 |
| formula 5 | 1.292 | 1.146 |
| formula 6 | 1.328 | 1.146 |
| formula 7 | 1.399 | 1.309 |

The other steroidal glycosides peaks eluting after 15 minutes have a response factor of 1.081 vs. compound of formula (2).

The result for various holding times is set out in table 3 below.

**Table 3 : content (in weight % based on anhydrous plant material) of steroidal glycosides of formulas (2) to (8).**

| Holding time | (2) | (8) | (3) | (4) | (5) | (6) | (7) | Other Ster. Glyc. | Total (2)-(8) |
|---|---|---|---|---|---|---|---|---|---|
| | | | | | | | | | |
| 0 | 0.086 | 0.040 | 0.064 | 0.052 | 0.055 | 0.026 | 0.012 | 1.061 | 0.335 |
| 4 | 0.105 | 0.045 | 0.083 | 0.057 | 0.073 | 0.043 | 0.016 | 0.809 | 0.423 |
| 12 | 0.104 | 0.042 | 0.081 | 0.057 | 0.074 | 0.042 | 0.016 | 0.784 | 0.417 |
| 16 | 0.100 | 0.041 | 0.080 | 0.051 | 0.075 | 0.045 | 0.016 | 0.772 | 0.408 |

As can be seen, by holding the harvested cut plant material before (reasonably quick) drying in accordance with the invention, the level of some of the identified actives (2) to (8) is higher after holding, and of the ones which are not much higher, they are at least not lower. Also, the level of "other steroidal glycosides" (i.e. the ones which are not identified and not proven to be effective in appetite suppression) goes down.

## Claims

1. Process for obtaining dried plant material from the *Apocynaceae* family comprising the steps of:
(a) removing the plants from the soil,
(b) cutting up the plants to pieces,
(c) holding the cut up plants for 1 - 36 hours, preferably 2-24 hours, at a temperature of 15-40°C wherein weight loss during this holding step is 0-10% of the fresh cut material,
(d) drying the so-obtained cut up plants, at a temperature of 40-120°C for 0.5 to 12 hours to arrive at a moisture content of below 10% (by weight).

2. Process according to claim 1, wherein the holding under (c) is done for 2-24 hours.

3. Process according to claim 1 or 2, wherein the drying of the cut up plants in step (d) is done at a temperature of 60-100°C, preferably at 70-90°C.

4. Process according to any of claims 1 to 3, wherein the drying step (d) is done for 1-8 hours, preferably for 1 to 5 hours.

5. Process according to any of claims 1 to 4, wherein the drying of step (d) results in a total moisture content of 0.5-10% by weight, preferably 1-8%.

6. Process according to any of claims 1 to 5, wherein after harvesting the roots of the plants are removed from the plant prior to or simultaneously with cutting up the plants in step (b).

7. Process according to any of claims 1 to 6, wherein the holding step (c) is carried out in the shade.

8. Process according to any of claims 1 to 7, wherein the process further comprises a step (e) after (d), comprising the step of obtaining a fraction of the dried material which comprises compounds of: formula (2) in a (mean) amount of 6-12%, formula (3) in a (mean) amount of 5-10%, formula (4) in a (mean) amount of 4-8%, formula (5) in a (mean) amount of 4-9%, formula (6) in a (mean) amount of 2-9%, formula (7) in a (mean) amount of 1-3%, formula (8) in a (mean) amount of 2-5%, after extraction of the dried plant material.

9. Process according to any of claims 1 to 8, wherein the temperature during holding step (c) is 20-35°C.

10. Process according to any of claims 1 to 9, wherein the plants are selected from the group consisting of *Hoodia gordonii, Hoodia currorii, Hoodia Iugardii* and mixtures thereof.

11. Process according to claim 10, wherein the plant is *Hoodia gordonii.*

12. Process according to any of claim 1 to 11, wherein of the pieces obtained by the cutting in step (b) one of the dimensions is less then 30 mm, preferably less then 20 mm, most preferably less then 15 mm.

13. Process according to any of claims 1 to 12, wherein the dried plant material further comprises one or more steroidal glycosides having the formulas (2) to (8) and mixtures thereof (Me=CH₃) :

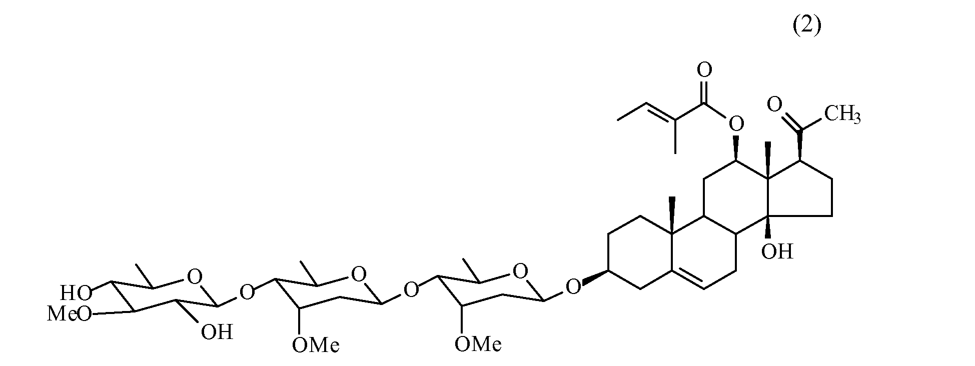

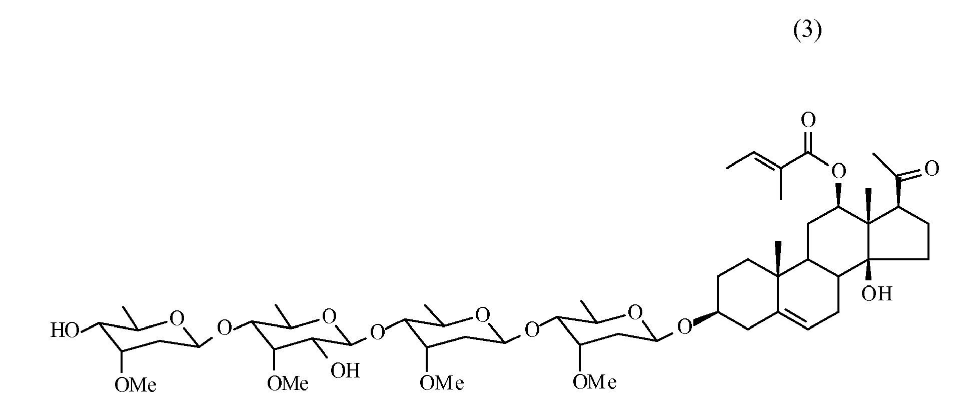

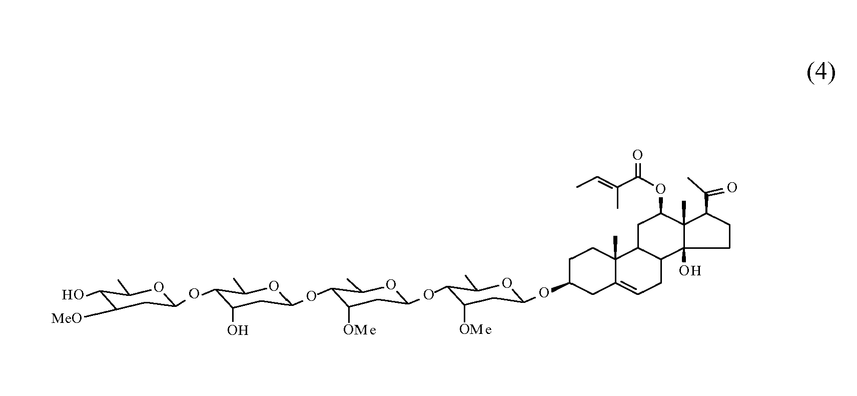

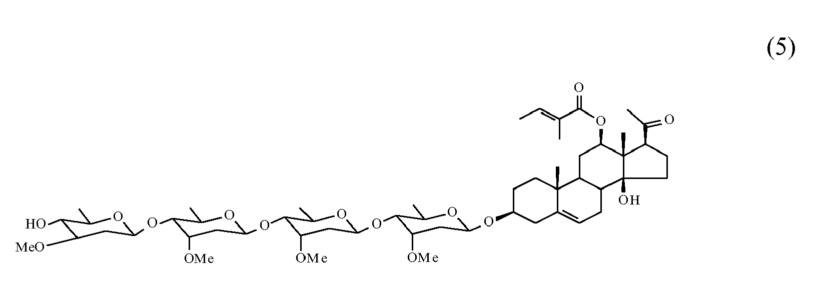

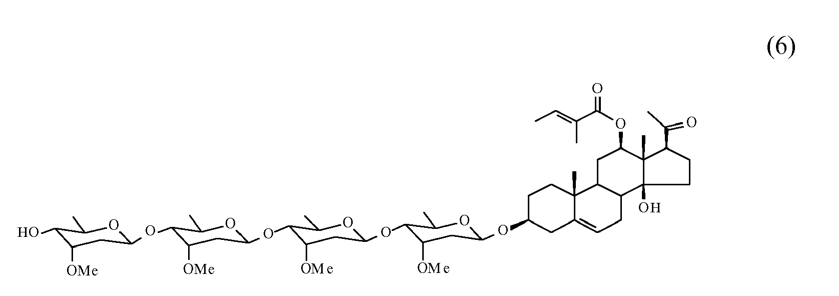

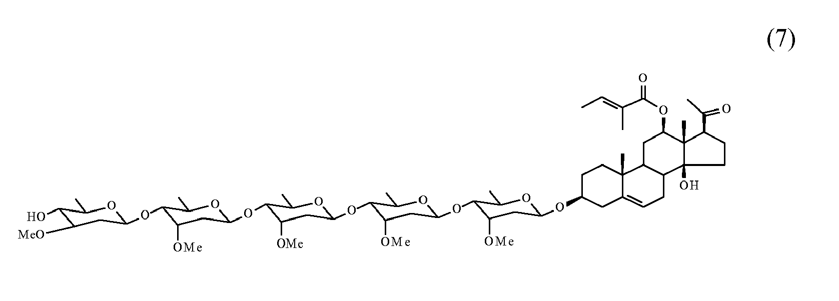

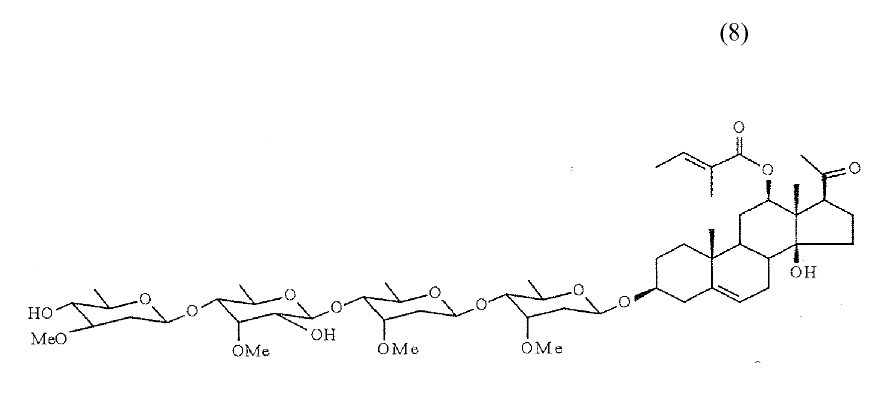

14. Process according to any of claims 1 to 13, wherein the dried plant material has a mean steroidal glycoside content of compounds (2) to (8) taken together of at least about 0.35% by weight, preferably at least 0.4%, based on anhydrous dried plant material.

15. Dried plant material obtainable by the process according to any of claims 1 to 14, wherein at least one of the steroidal glycosides (2) to (8) is increased by at least 20% in weight compared to a plant material which is prepared by a process without step (c), and which dried plant material has a moisture content of below 10% by weight.

16. Dried plant material according to claim 15, wherein it comprises the compound of formula (2) in a mean amount of at least 0.095% by weight, based on anhydrous dried plant material.

17. Composition comprising one or more of the steroidal glycosides of formulas (2) to (8) and other steroidal glycosides, wherein the (mean) weight ratio between the steroidal glycosides of formulas (2) to (8) (when taken together) to other steroidal glycosides is at least 0.4, more preferably said ratio is at least 0.45.

18. Composition according to claim 17, wherein such composition is dried, comminuted plant material.
